# EUROPEAN PATENT APPLICATION

(11) **EP 3 965 013 A1**
(43) Date of publication of application: **09.03.2022**
(21) Application number: 20195160.5
(22) Date of filing: 08.09.2020
(51) Int. Cl.: G06N 3/04, G06N 3/08, G06N 5/04

(54) **MACHINE LEARNING CLASSIFIER USING META-DATA**

(71) Applicant: Koninklijke Philips N.V., 5656 AG Eindhoven (NL)
(72) Inventor: Vdovjak, Richard, 5656 AE Eindhoven (NL)
(74) Representative: Philips Intellectual Property & Standards

(57) **Abstract**

Some embodiments are directed to training method a classifier. The classifier receives sensor data as input and produces a label as output. A quality estimator is applied to meta-data of a training sample, obtaining a quality estimation of a ground-truth label of the training sample. The classifier may be trained on the training sample taking into account the quality of the ground-truth label.

## Description

### FIELD

The presently disclosed subject matter relates to a training method for a classifier, a system for training a classifier, a system for applying a classifier, a workstation, an imaging apparatus, and a computer readable medium.

### BACKGROUND

As noted in the FDA discussion paper "Proposed Regulatory Framework for Modifications to Artificial Intelligence/Machine Learning (AI/ML)-Based Software as a Medical Device (SaMD)", Artificial intelligence (AI)- and machine learning (ML)-based technologies have the potential to transform healthcare by deriving new and important insights from the vast amount of data generated during the delivery of healthcare every day. Example high-value applications include earlier disease detection, more accurate diagnosis, identification of new observations or patterns on human physiology, and development of personalized diagnostics and therapeutics. One of the greatest benefits of AI/ML in software resides in its ability to learn from real-world use and experience, and its capability to improve its performance. The ability for AI/ML software to learn from real-world feedback (training) and improve its performance (adaptation) makes these technologies uniquely situated among software as a medical device (SaMD) and a rapidly expanding area of research and development. The FDA's vision is that with appropriately tailored regulatory oversight, AI/ML-based SaMD will deliver safe and effective software functionality that improves the quality of care that patients receive.

One application of deep-learning neural network image classifiers is to interpret radiographs. For example, the image classifier may receive as input a radiograph and produce as output a classification of the image, e.g., the presence or absence of an abnormality, or a classification of the type of abnormality, if any. Such a classifier may assist radiologist in interpreting radiographs.

A known system is disclosed in the paper 'MURA: Large Dataset for Abnormality Detection in Musculoskeletal Radiographs' by Pranav Rajpurkar, et al. The known system is trained for recognizing abnormalities in upper extremity abnormalities, e.g., finger, wrist, humerus, and so on. For example, upper extremity abnormalities that may be classified from a radiograph include fractures; hardware, e.g., a forearm showing a screw fixation and/or an operative plate; degenerative joint diseases; lesions; subluxations, and so on. The known model groups all abnormalities in a single group. However, it would also be possible to classify the individual type of abnormality.

The known model is a feed-forward 169-layer convolutional neural network that outputs a probability of abnormality when provided with a radiograph as input. If multiple views are available an overall probability of abnormality is determined as the arithmetic mean of the abnormality probabilities output by the network for each image. Input images are scaled and normalized.

Before training, the weights of the network were initialized with weights from a model pretrained on ImageNet. During training of the network an initial learning rate is decayed each time the validation loss plateaus after an epoch.

The known model was trained on a dataset containing 14,863 musculoskeletal studies of the upper extremity, where each study contains one or more views and was manually labelled by radiologists as either normal or abnormal at the time of diagnostic clinical radiographic interpretation.

To evaluate the model and to compare to radiologist performance, six additional normal/abnormal labels were collected from six radiologists on a holdout test set of 207 studies. These radiologists had experience ranging from 2 to 25 years. Three radiologists were randomly chosen and a majority vote of their labels was taken as the gold standard. The labels of the other three radiologists were used to get estimates of radiologist performance on the task.

Interestingly, agreement among the radiologists was sometimes surprisingly low. For example, agreement on radiographs of a finger gave a Cohen's kappa between 0.3 and 0.41. That is, there was little agreement between the labels of the three individual radiologists and the gold-standard majority vote of three other radiologists. The model also did not fare well on finger studies either with a kappa of 0.389. On the other hand, for wrist studies, there was good agreement between radiologist with a kappa between 0.79 and 0.93. The model fared also much better with a kappa of 0.71

The traditional approach to ground-truth labels is not ideal. Agreement between radiologist is sometimes low, even among a small group of six radiologists assessing a small test set under test conditions. The quality of the ground-truth labels obtained from many more radiologist taken under a range of conditions will not be much better. The inventor realized that not all experts are able to deliver quality labels at the same level and consistency rate. Moreover, the number of human errors will differ depending on the time of the day, day of the week, and potentially many other factors. At the same time, the ground-truth quality has an impact on the overall model performance.

### SUMMARY

The problem of training machine-learnable models with ground-truth data of varying quality is common to many applications. Medical classification of images being one example. It would be advantageous to have an improved method for training a classifier. In particular, it would be advantageous to have an improved method for training an image classifier, e.g., a medical image classifier, the ground-truth label indicating a medical abnormality in the image. It is an object of another aspect of the invention to provide medical imaging devices that are better able to assist in reaching the correct diagnosis. The invention is defined by the independent claims. The dependent claims define advantageous embodiments.

The background example shows that the noise in ground-truth label can vary significantly. The inventor realized that ground-truth labels are often noisy, but that the noise is not uniform. Some sensor data has a higher chance of having an incorrect label than others. Interestingly, sensor data that are more or less likely to have an incorrect label can be identified from information associated with the sensor data, e.g., the so-called meta-data. The meta indicates information on the origin of the ground-truth label. The meta-data can be used to approximate which sensor data are more or less likely to have correct ground-truth labels. This information can be used to train a classifier less aggressively on the noisier labels, e.g., a lower learning rate, or a lower priority. Although a classifier, especially one based on a neural network, is capable of learning in spite of noise, the learning will be faster and the end-result more accurate if noise is taken into account during training. Some percentage of the sensor data that has the noisiest labels can be ignored during training altogether.

Distinguishing among ground-truth labels in training a machine learnable model on the basis of quality provides advantages. For example, the quality of the machine learnable model improves, training is less susceptible to noise and training may proceed faster. There are various ways to embed quality measures into the machine learning workflow. A good example is to modulate the learning rate with a quality measure-so that high quality training samples have more impact on the trained machine learnable model. Another approach is to prioritize high quality training samples, e.g., include them in training iterations more often. Low quality training samples may be excluded from training altogether.

Methods and systems are proposed that use meta-data associated with ground truth data, e.g., ground-truth labels. Such methods and systems may be applied in a machine learning task where the ground truth is produced by humans, e.g., domain experts. In the latter situation, the quality of ground-truth labels will vary, e.g., due to different levels of expertise, experience, timing, and so on. A particular important application is image classification, especially medical image classification. Other image classification tasks in which an embodiment may be applied are indicated herein.

The classifier may be applied to sensor data. For example, sensor data may be obtained from a sensor device. The sensor device may give technical data regarding a physical object in the environment of the sensor device. The object may be a machine, e.g., indicating a technical state of the machine, a traffic scene, e.g., indicating traffic signs, vehicles and the like. An object may be a human or animal body, e.g., indicating a medical aspect of the body. A good example of sensor data is an image which may be obtained from an imaging device.

For example, a quality estimator may translate meta-data in a numerical value indicating the quality of the ground-truth label or labels. The quality estimates may be rule based. For example, results of studies on radiological performance may be encoded into rules. An advantage of a rule-based system is ease of modification, and high explainability. The quality estimator may comprise a machine learnable model, trained on pairs of meta-data and quality scores. The latter may be determined by domain experts, and/or may be automatically generated. A workstation or imaging apparatus, e.g., a medical workstation or imaging apparatus may be configured to apply an embodiment of the training method.

Once trained, the classifier may be applied to novel sensor data, e.g., obtained from a sensor device. The sensor data may comprise an image obtained from an image device.

In an embodiment, the classifier may be fine-tuned, e.g., further trained using sensor data, e.g. images and labels obtained during the use-phase. An aspect disclosed herein is a system for training a classifier, and a system for applying a classifier. A system and/or device configured for training a classifier is an electronic system and/or device. For example, the system may be a computer.

The training method described herein may be applied in a wide range of practical applications. Such practical applications include training image classifiers to assist medial decision makers, for quality control, or in other image classifiers, e.g., to recognize objects in road scenes.

A person skilled in the art will appreciate that the method may be applied to multi-dimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

These and other aspects of the invention will be apparent from and elucidated with reference to the embodiments described hereinafter.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of an embodiment of a system for training an image classifier,
Figure 2 schematically shows an example of an embodiment of a system for training an image classifier,
Figure 3 schematically shows an example of an embodiment of a system for applying an image classifier,
Figure 4 schematically shows an example of an embodiment of a quality estimator,
Figure 5 schematically shows an example of an embodiment of a system for training a quality estimator,
Figure 6 schematically shows an example of an embodiment of a method for training an image classifier,
Figure 7a schematically shows computer readable media,
Figure 7b schematically shows a representation of a processor system according to an embodiment.

### List of Reference Numerals:

- 110: a system for training an image classifier,
- 130: a processor system
- 140: a storage
- 150: communication interface
- 200: a system for training an image classifier,
- 210: an image classifier storage
- 220: a training storage
- 230: a training sample
- 231: a training image
- 232: a ground-truth label
- 233: meta-data
- 240: a quality estimator
- 241: a quality estimate
- 250: an image classifier
- 251-253: a determined label
- 255: an image classifier
- 260: a machine learning part
- 270: an imaging device
- 271: an image
- 272: a display
- 300: a system for applying an image classifier
- 400: quality estimator
- 411: a default quality estimate
- 412: a quality estimate
- 421-423: a quality estimate modifier
- 430: a meta-data
- 431-433: meta-data attributes
- 500: a system for training a quality estimator
- 510: a comparator
- 511: a determined quality estimate
- 540: a machine learning part
- 560: a machine learning quality estimator
- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as feature described herein or recited in mutually different dependent claims may be combined.

Known machine learning approaches treat the labels of training data-the ground-truth (GT)-both uniformly and opaquely. Uniformly in the sense of the same truth/correctness expectations along all labeled samples. Opaquely in the sense of not utilizing and typically not even aware of the metadata information associated with the individual labeled training samples, such as who created the label, when, where, under what conditions, etc.

Most AI approaches, and deep learning approaches in particular, are able to cope with some noise in the ground truth data, e.g., incorrect labels, to some extent. It was found though that ground-truth quality has a positive impact on model performance. In particular, once the AI model reaches the performance of the domain expert, it becomes increasingly hard to improve, precisely because it needs to substantially overcome the noise levels.

In embodiments, a system and a method are provided to treat ground truth labels individually, and to exploit the relevant domain-specific metadata. For example, in an embodiment, a ground-truth quality score is obtained for each sample, e.g., computed using a further model. The ground-truth quality score may be utilized in improved training of the image classifier. Embodiments are also useful for in-product learning environments. In an in-product learning environment, the images and associated ground-truth labels assigned by domain experts are collected, e.g., on a daily or weekly basis, and used for further training of the image classifier. This has the appeal that the image classifier improves while it is used. The ground-truth label(s) are typically produced by many domain experts with various expertise level in such a setting. Using quality estimates can improve the further learning that can be obtained from in-product learning. Improved learning from noisy GT labels increases the quality of resulting image classifier, which in turn helps the adoption of AI in healthcare.

Embodiments use classification of images as a motivating example; however, the method may be applied to training machine learnable models on any domain data, e.g., sensor data, which is labeled by domain experts. For example, sensor data may comprise a collection of sensor readings, e.g., temperature, pressure and the like, measured at multiple points in a machine, e.g., a motor, an industrial plant, etc. Image data could be part of the sensor data, but this is not needed. Ground-truth labels may indicate whether a sensor data, e.g., a collection of sensor data items, are abnormal or not, e.g., are safe or not. Other labels or classifications are possible. Ground truth data may indicate a particular problem. For example, such training data may be collected by recording said sensor data together with information that indicates if a human operator intervened in the system or not. Given sensor data, the trained model will be able to predict whether an intervention is warranted. Although obtaining large amounts of training data is thus possible, the same problem occurs as with medical assessments. Some human operators have little experience, less training or are less reliable for other reasons, e.g., time of day. By using quality estimates their intervention decisions may be made to carry less weight in training. For example, embodiments described below for images may be directly modified to other sensor data.

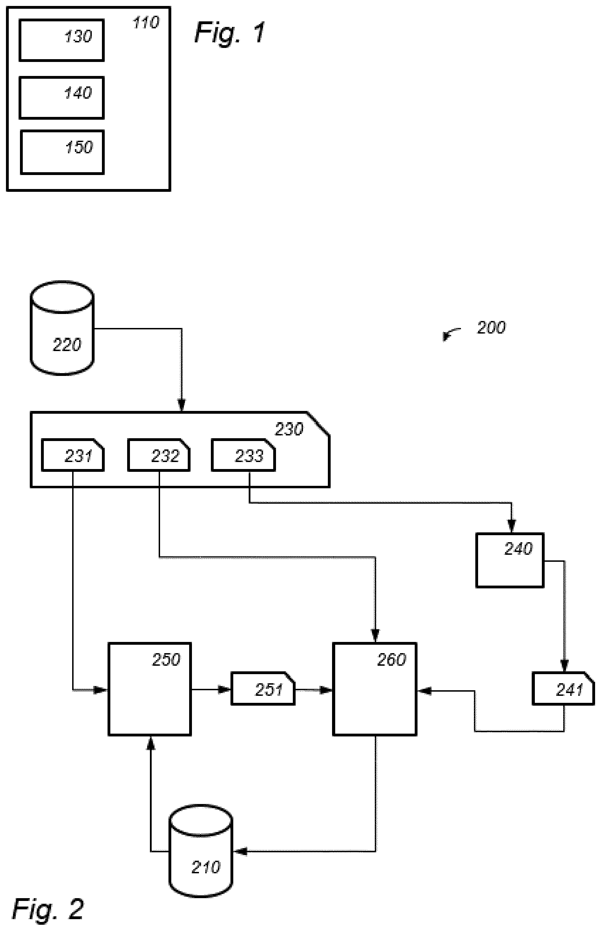

**Figure 1** schematically shows an example of an embodiment of a system 110 for training an image classifier. For example, the system 110 of figure 1 may be used to train an image classifier. System 110 may also be configured to evaluate the image classifier. System 110 may comprise a processor system 130, a storage 140, and a communication interface 150.

Storage 140 may comprise local storage, e.g., a local hard drive or electronic memory. Storage 140 may comprise non-local storage, e.g., cloud storage. In the latter case, storage 140 may comprise a storage interface to the non-local storage. For example, the storage may comprise image data, e.g., radiograph image data, training data, model parameters, and so on.

System 110 may communicate internally, with other systems, external storage, input devices, output devices, imaging devices, and so on over a computer network. The computer network may be an internet, an intranet, a LAN, a WLAN, etc. The computer network may be the Internet. The system comprises a connection interface which is arranged to communicate within the system or outside the system as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. Internal and/or external communication may also use a proprietary communication protocol, e.g., a bus.

In system 110, the communication interface 150 may be used to send or receive digital data. For example, communication interface 150 may be used to receive an input image for providing the image to the image classifier, and/or to transmit an output of the image classifier, e.g., one more labels, e.g., to a display, a user of the system, etc. For example, communication interface 150 may be used to send and/or receive model parameters, training data, etc.

The execution of system 110 may be implemented in a processor system, e.g., one or more processor circuits, e.g., microprocessors, examples of which are shown herein. System 110 may comprise multiple processors, which may be distributed over different locations. For example, system 110 may use cloud computing.

Other figures show, inter alia, functional units that may be functional units of the processor system. For example, figure 2 may be used as a blueprint of a possible functional organization of the processor system. The processor circuit(s) are not shown separate from the units in these figures. For example, the functional units shown in figure 2 and the like may be wholly or partially implemented in computer instructions that are stored at system 110, e.g., in an electronic memory of system 110, and are executable by a microprocessor of system 110. In hybrid embodiments, functional units are implemented partially in hardware, e.g., as coprocessors, e.g., neural network coprocessors, and partially in software stored and executed on system 110. Part or all of system 110 may be implemented in a single device.

**Figure 2** schematically shows an example of an embodiment of a system 200 for training an image classifier. For example, system 200 may be implemented on a system such as system 110. For example, system 200 may be implemented on a computer, either a single computer or multiple computers, e.g., a distributed computing system. System 200 may be implemented in a workstation or imaging apparatus. System 200 may be implemented in a server, e.g., accessible over a computer network, such as the Internet. Other examples are disclosed herein.

Shown in figure 2, is an image classifier 250. Image classifier 250 is configured to take at least an image as input to classify the image. For example, image classifier may comprise a machine learnable model to classify the image. Typically, image classifier 250 comprises a neural network. For example, the image classifier may be configured to output a label classifying the image. For example, the image classifier may have one or multiple output nodes, that produce a likelihood, e.g., a probability, that a particular label applies to the image. A useful image classifier might have only a single output, e.g., normal/abnormal. For example, a value between 0 and 1 may be generated indicating a likelihood that the image is abnormal. The image classifier may have multiple outputs, e.g., indicating which of multiple abnormalities applies to the input image. The image classifier may comprise multiple layers, including, e.g., one or more convolutional layers, ReLu layers, pooling layers, fully-connected layers, etc. For example, the model may be a feed-forward network. For example, the network may be of the design described in the background.

Embodiments may be applied to different image classification domains; associating a ground-truth quality score per training sample is applicable across various domains. For example, the image classifier in an embodiment may be configured for face recognition, visual geolocation, gesture recognition, object recognition, driver assistance, e.g., identifying objects on the road, image tagging, quality control, e.g., recognizing a defect in an image of manufactured product, and so on. A particular important application, however, are medical image classifiers. Not only are medical image classifiers important in their own right, getting an accurate output is especially important for this application. Moreover, the problem of varying quality of ground-truth labels, in a way that can be predicted from meta-data was observed in this particular setting. For example, in the context of quality control, an image classifier can be trained on previously

For example, in an embodiment, the image classifier is a medical image classifier. For example, the ground-truth label may indicate a medical abnormality in the image.

The image may be obtained from various types of imaging devices. For example, the imaging device may be a camera, e.g., a visible light camera, an infrared camera, etc. For example, an image classifier configured to recognize traffic signs in images may use a regular camera, e.g., sensitive for visible light.

The imaging device may also be configured for other modalities, e.g., in the field of medical image classification. For example, in an embodiment the image may be multi-dimensional image data, e.g., to two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

For example, in an embodiment, the images may be radiographs of upper extremities. The ground-truth labels may indicate if a medical abnormality is visible in the image, e.g., as described in the background. For example, heart problems may be diagnosed from an MRI image, etc.

Figure 2 shows an image classifier storage 210. Storage 210 stores weights that characterize the image classifier; for example, these may be the weights that configure the neural nodes in a neural network. Before training, the weights for the image classifier may be initialized. This may be done randomly, or taken from a pre-trained network, etc. The network weights may be pre-trained in part and randomized in part, e.g., randomized in one or more final layers.

Figure 2 shows a training storage 220 comprising multiple training samples. One training sample 230 is shown. A training sample comprises: a training image 231, a ground-truth label 232 and meta-data 233. The training of the image classifier is configured so that image classifier learns to reproduce a ground-truth label 232 when given the training image 231. The training image 231 may be as indicated herein, e.g., a radiograph, MRI image, etc. Typically, the ground-truth labels are produced by domain-experts. For example, the ground-truth label may be produced by a domain-expert who judges, or reads, the training-image. For example, training data may be obtained by having one or more domain experts judging a collection of training images to provide the ground-truth labels. For medical images, a good source of ground-truth data is to obtain them from domain experts as they supply their diagnosis of the image; for example, ground-truth data may be extracted from reports written by domain-experts concerning one or more images.

For example, in the case of radiographs, the ground-truth labels may be produced by radiologists. Unfortunately, the ground truth labels may be noisy. For example, some domain experts may be less accurate than others. For example, a domain expert may be more accurate at some times of the day or week than others, or may be more accurate for some topics than for others.

Meta-data 233 provides information on the origin of the ground-truth label. Meta-data may comprise various types of information. For example, one type of information may relate to the domain-expert him- or herself. For example, meta-data relating to the domain expert may comprise one or more of: the domain expert identifier, the domain expert's specialty and/or the domain expert's subspecialty, the domain expert's years of experience, case volume, number of past cases, etc.

For example, one type of information may relate to the creation of the ground-truth label. For example, meta-data relating to the creation of the ground-truth label may comprise one or more of: a timestamp when the label was created, the duration of creation, (sub-)specialties associated with ground-truth label or the report in which it is reported, a creation location, etc.

For example, one type of information may relate to the image and/or exam for which the ground-truth label was created. For example, meta-data relating to the image and/or exam may comprise one or more of: an image modality, an exam type, a clinical need for the image.

For example, a label may be the presence or absence of a medical abnormality. Medical labels, in particular the presence or absence of an abnormality may be extracted from radiology reports. Such labels may be curated by hand, or they may be collected automatically. For example, reports may be created by filling in a template or the like in which the labels may be entered in a label field. The label or labels and meta-data may be extracted from the template by a computer program.

Interestingly, labels and/or meta-data may be extracted from a domain expert report such as a medical radiologist report using a further AI model, e.g., a natural language processing (NLP) model. For example, the NLP model may extract the domain specific metadata and/or GT label(s) for every data sample and store it together with the image.

For example, an extraction system may be presented with multiple reports of domain experts associated with one or more images. The extraction system may be configured to collect ground-truth labels and meta-data from various sources. For example, ground-truth labels and meta-data may be extracted from a report. For example, meta-data may be extracted from system information, e.g., file creation data, total editing time, etc. The extracting system extracts the ground-truth labels and metadata obtained from multiple reports and store it together with the image as a training sample. Training data may also be curated by hand, or using a partially automated system. The use of an NLP model to extract labels of meta-data, although convenient, is not needed.

Note that the inventor found that metadata could also be used directly by an image classifier, e.g., as additional features included in the input together with the input image. For example, instead of or in addition to using quality measures to manage the training process, it is possible to configure the machine learnable classifier to receive quality correlated inputs directly. For example, quality, either as a combined score, or even as a number of individual sub-scores per measured quality dimension, may be used in training as explicit input features, e.g., next to the problem domain data, e.g., imaging data. This has the advantage that the machine learning can pick up on the optimal combination. Validation can be done, e.g., against a high-quality domain expert.

Image classifier 250 may be trained with a machine learning training algorithm appropriate for the type of image classifier. System 200 may comprise a machine learning part 260 configured with the machine learning training algorithm.

For example, the machine learning training algorithm may be based on gradient descend. For example, neural network based image classifiers may be trained using a gradient descend training algorithm. For example, one may employ the Adams training suite. For example, to train image classifier 250 one may iteratively obtain a training sample or batch of training samples from the training storage and apply the machine learning training algorithm. Typically, one applies the image classifier 250 to a training sample and then modifies the parameters, e.g., weights of the image classifier 250 so that its output will be closer to the desired output, e.g., close to the ground-truth label.

Unfortunately, the quality of ground-truth labels may sometimes be higher and sometimes lower. System 200 comprises a quality estimator 240. Quality estimator 240 is configured to apply a quality estimation function to meta-data 233 of the training sample 230, thus obtaining a quality estimation 241 of the ground-truth label 232 of the training sample. For example, the quality estimator 240 may provide a numerical estimate. There are various ways to configure the quality estimation, examples of which are provided herein.

The quality estimation 241 may be used to reduce the negative impact of potential noisy data on the training of image classifier 250. For example, system 200, e.g., machine learning training part 260 or quality estimator 240 may determine a learning rate from the quality estimation. For example, the determination may be a look-up table, a function, an algorithm or the like. The quality estimation may be directly expressed as a learning rate. For example, a training sample with a high quality estimate may be use with a high learning rate, while a training sample with a lower quality estimate may be used with a lower learning rate. Note that, instead of modulating the learning rate with the quality estimation, the quality estimation could be used in other ways, e.g., prioritizing training samples, removing low quality samples, etc.

For example, a quality estimation may be expressed as a number, e.g., a floating-point number, e.g., between 0 and 1, between -1 and +1. For example, a quality estimation may be a value, around 1, for example, lower estimated quality may correspond to values below one, and higher estimated quality corresponding to values above one. A default learning rate may be multiplied with the quality estimation. This approach has the advantage that conventional learning rate schedule may be used, adapted by the quality estimates. For example, learning rates may decrease according to the schedule, say a decrease in subsequent epochs. A given learning rate may be multiplied with the quality estimate. A function may be applied to a quality estimate to obtain the learning rate. The function may be a look-up table, e.g., mapping quality estimate ranges to a learning rate.

For example, quality estimates may be discrete, e.g., binned in various classes, e.g., very low, low, average, high, very high. Determining a learning rate may also be done by looking up a learning rate in a table given the quality estimate. A different table may be used for different epochs.

The machine learning part 260, e.g., the machine learning algorithm implemented by it, may be configured to take as input the learning rate. For example, a magnitude of the modification depending on the learning rate. For example, in case of gradient descend, a derivative of the error may be multiplied with the learning rate.

For example, machine learning part 260 may take as input a current set of weights, e.g., as stored in storage 210, and a training image 231, a determined label 251, e.g., the label produced for the current training image using the current set of weights, a ground-truth label 232 for the image, and a learning rate. The machine learning part 260 is configured to determine a new set of weights so that an image classifier with the new set of weight approximates the ground-truth label closer. The size of the modifications made to the weights may depend on the size of the error, but in particular also depend on the size of the learning rate.

An advantage of modulating learning rate in dependency on the quality estimate is that noisy estimates can still be used in training and need not be discarded, at least not all of them. Even if noise is present, noisy data still contains useful information, by modulating the learning rate the noisier data can still be used, but more trustworthy data can be given a higher significance.

In an embodiment, a quality score may be used, e.g., in the context of neural network based models, to adjust the learning rate, e.g., a hyper parameter that influences to what extent the optimizer adjusts the weights in the model such that they approximate better the desired results. Lowering this rate for ground-truth samples with lower quality means that the model learns from them, though to a lesser extent than those with a high quality score. In colloquial terms, the model still learns from all samples, but samples with a low quality score are taken with a grain of salt. This enables one to effectively utilize all data samples but to learn more from those with a higher quality score.

In an embodiment, the quality estimate may be used to filter the training data. For example, training samples with a quality estimate below a threshold may not be taken into account. In an embodiment, training samples with a high quality estimation are prioritized over training samples with a lower quality estimation. For example, a training sample with a higher quality estimate may be used in more training iterations than one with a lower quality estimate. Filtering the training data on quality estimates may be done with or without modulating the learning rate from a quality estimates.

In an embodiment, training may be batched on multiple images with a similar quality estimates, which may use a similar learning rate. For example, in an embodiment, system 200 is configured to determine the quality estimate first for multiple training samples from the training storage. From the multiple one or more batches may be selected having a similar quality estimate. For example, all or part of the training samples may be binned in dependence upon the quality estimate. Machine learning part 260 may be configured to process a batch using the same learning rate. Batched training is more efficient, and is compatible with using quality estimates.

For example, in an embodiment, multiple training samples may be stored in a training storage 2220. A quality estimator 240 may determine a quality estimate for the training samples. Image classifier may be configured to compute a determined label for a training image. A machine learning part may modify the image classifier weights to improve the determined label, wherein high-quality training samples are given more weight than low-quality training samples.

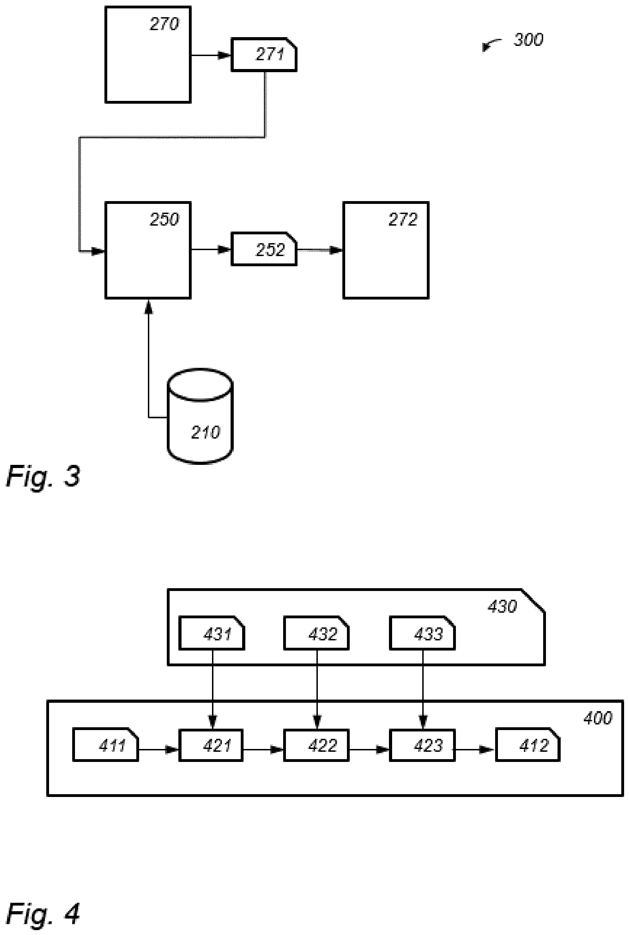

**Figure 3** schematically shows an example of an embodiment of a system 300 for applying an image classifier. System 300 may also be implemented on a system such as shown in figure 1. Once the image classifier has been trained, the image classifier may be applied. System 300 for applying an image classifier may be connected to an imaging device 270 to receive an image 271. For example, device 270 may be a radiography device for obtaining X-ray images. For example, device 270 may be an MRI device.

System 300 may comprise an image classifier 250, e.g., trained by an embodiment. System 300 may be configured to receive image 271 and provide it to the image classifier 250 to obtain a determined label 252. System 300 may comprise a display or the like to display the label 252, possibly in conjunction with image 271. Image 271 is typically a novel image, e.g., an image that image classifier 250 has never seen in training.

System 300 may use a computer network to receive image 271 from imaging device 270, and/or may use a computer network to deliver the image 271 and determined label 252 to a display.

Note that system 300 does not require quality estimator 240 or machine learning part 260. These parts may be discarded one the training is complete. On the other hand, they may also be retained; For example, a system, e.g., system 110, may be configured for both training and usage. For example, having quality estimator 240 or machine learning part 260 has the advantage that further fine-tuning of the model may be done locally, e.g., after deploying image classifier 250.

For example, an image classifier may have a training phase in which it is configured and a usage phase in which it is deployed. However, it is possible to switch back and forth between these phases. For example, one may train a model, then use it on novel images, and later return to training for additional training, e.g., fine-tuning of the model. System 300, with or without training facilities may be included in a workstation or imaging apparatus, and the like.

For example, if local additional training is done, one may obtain meta-data and a label for the novel image. For example, the label may be a ground-truth label from a domain expert. The novel image and label may be used for fine-tuning. Interestingly, an embodiment may be done for the additional training as well, for example, a quality estimate may be obtained as in an embodiment, which in turn may be used to adapt the training, e.g., change the learning rate.

Performance of domain experts is not consistent throughout the day, but subject to diurnal and circadian rhythms. During the day performance changes. Moreover, it is known that there is a temporary drop in performance after lunch. In addition to time of day, many other factors have been shown to contribute to the likelihood of incorrect ground-truth labels, these include, for example: the radiologist's level of experience, and fatigue, e.g., the number of consecutive hours an individual has been awake, caseload, and case transition. The review paper "Effects of time of day on radiological interpretation", by A.S. Alshabibi, M.E. Suleiman, K.A. Tapia, P.C. Brennan studies the accurate interpretation of radiological images with a view to improve patient outcomes by developing reading protocols for radiologist. The paper gives an overview of some of the factors that are known to impact radiologist performance.

In an embodiment, the quality estimator summarizes these factors into a quality estimate, e.g., a numerical estimate that indicates the likelihood that the ground-truth label is correct. For example, if the training samples were portioned into a set with low quality estimates and one with high quality estimates then less noise would be expected in the ground-truth labels in the second set.

A quality estimator may be implemented in various ways. For example, in an embodiment, the quality estimator may apply a set of rules to the meta-data to compute the quality estimate. For example, favorable elements according to the rules, e.g., high experience, low fatigue, may lead to a higher quality score, while unfavorable elements, e.g., performing a reading just after lunch, may lead to a lower quality score.

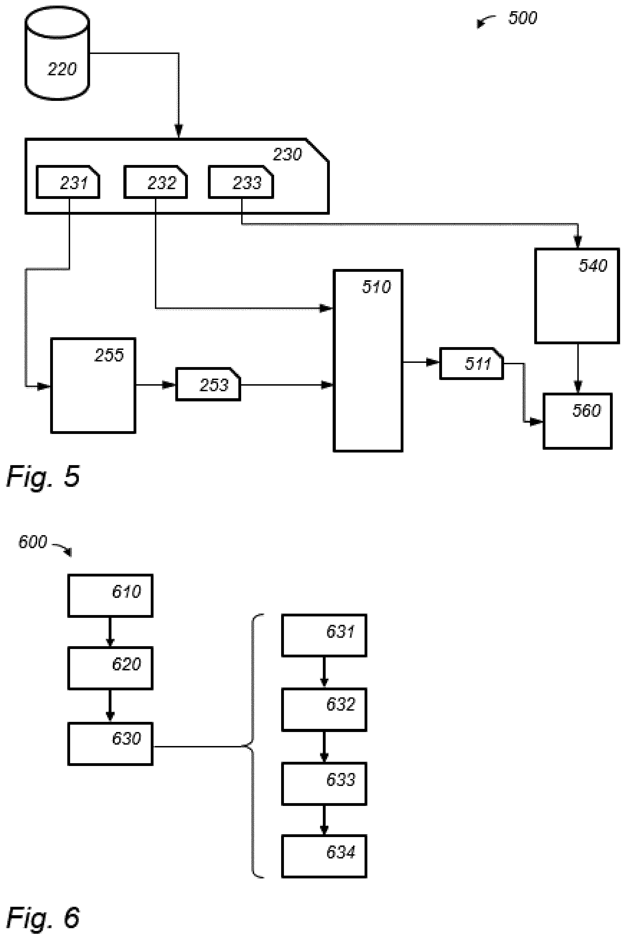

For example, in an embodiment, a time of day may be mapped to a quality estimate, e.g., using a look-up table, possibly further modified using other meta-data elements. Two further quality estimator embodiments are described with respect to figures 4 and figure 5.

**Figure 4** schematically shows an example of an embodiment of a quality estimator 400. Figure 4 shows meta-data 430 comprising meta-data attributes 431-433. For example, meta-data attribute 431 may indicate the time of day the ground-truth was evaluated by the domain-expert. For example, meta-data attribute 432 may indicate whether the (sub)specialty of the domain expert matches the domain of the image. For example, meta-data attribute 432 may be positive if the image is an image of a finger while the domain expert has fingers as a specialty, and negative if not. For example, meta-data attribute 433 may indicate the number of hours the domain expert was awake when the reading was done. More, fewer, and/or different choices of meta-data attributes are possible, e.g., as summarized above.

Quality estimator may initialize a default quality estimate. For example, the default estimate may be set to 1, or 0, or the like. Quality estimator 400 may comprise quality estimate modifiers 421-423 that are configured to modify estimate 411, up or downward depending on finding positive or negative elements. The modifiers may be associated with particular meta-data elements, and modify the estimate 411 upwards or downwards.

For example, a reading taking just after lunch may receive a downward adjustment, while a reading taken by a domain expert many years of experience may receive an upward adjustment. For example, the modifies may be organized in a rule base, or a decision tree or the like. After all modification have been applied, a final quality estimate 412 is obtained. The modifier may multiply with a factor, or add or subtract from a quality estimate. For example, a modifier may add or subtract points to a quality score, where a higher score is likely to correspond to a higher quality ground-truth label. An advantage of this approach is that it can be easily modified by adding, removing or editing rules. The system is also robust if some particular meta-data is not available for a particular image, as the corresponding modifier could be skipped. Moreover, this approach has high explainability.

It should be noted that it is not necessary for a quality estimator to provide a perfect indication whether or not a ground-truth label is correct or not. It is sufficient that images can be stratified according to approximate quality.

**Figure 5** schematically shows an example of an embodiment of a system 500 for training a quality estimator. System 500 uses a trained image classifier to train a second model as a quality estimator.

System 500 uses an initial image classifier 255. Image classifier may be trained on the training set, e.g., stored in training storage 220. Image classifier 255 may be trained conventionally, e.g., without regard for the meta-data, e.g., without estimating ground-truth quality. Image classifier 255 may also be trained according to an embodiment, e.g., using a quality estimator according to figure 4, or some other embodiment. Image classifier 255 is preferably fully trained so that labels produced by it are a good approximation of expert classifications.

System 500 uses image classifier 255 to associate the training samples with a determined quality estimate. For example, consider training sample 230 comprising a training image 231, a ground-truth label 232 and meta-data 233. Image classifier 255 is applied to training image 231 to obtain a determined label 253. A comparator 510 compares the image classifier output 253 to the ground-truth label 232 to determine a determined quality estimate 511. For example, if the ground-truth label 232 and determined label 253 are each numerical values, e.g., between 0 and 1, then the determined quality estimate may be their squared difference. If multiple labels are output, then the determined quality estimate may be distance between the two vectors, e.g., a Euclidean distance or the like. Many other distance measures are known, e.g., L1, L2, L-infinity norm and so on.

Once a determined quality estimate is obtained for a sufficiently large number of training samples, possibly for all of them, the determined quality estimate may be used to train a quality estimator 560. For example, machine learning part 540 may be configured with a machine learning algorithm to train the quality estimator 560 to map meta-data to a determined quality estimate, e.g., to map meta-data 233 to determined quality estimate 511. Quality estimator 560 may comprise a neural network, but this is not necessary; For example, quality estimator 560 may comprise some other machine learning model, e.g., random forests.

The input to a machine learning quality estimator may be a vector that defines the meta-data. For example, components of the vector may indicate a value of a defined aspect of meta-data. For example, a component of a vector may indicate years of experience. For example, a component may indicate time of day, etc.

Once quality estimator is trained it can be used to train an image classifier, possibly even on the same training set used to train the quality estimator 560. The training may be applied to a new image classifier, e.g., to train classifier 250 from initialized weights, or may be used to fine-tune, e.g., retrain, additionally train, image classifier 255. Quality estimator 560 may also be used for in-product training. For example, an image classifier may be fine-tuned on images obtained during a usage phase.

To improve the robustness of quality estimator 560, its input may include data that is associated with the accuracy of the domain experts but not with the accuracy of image classifier 255. The input may exclude data that is correlated or correlated too much with the accuracy of image classifier 255. For example, if the specialty or the image itself were included, the quality estimator may learn in which fields the image classifier 255 underperforms rather than where the ground-truth labels are less accurate. On the other hand, it is not likely that, say, time of day, time of week, hours awake, and so on, are correlated to how well image classifier performs on corresponding image.

Instead of excluding meta-data which is correlated with the accuracy of the image classifier, other approaches of improving the quality estimator are also possible. For example, a quality estimate may be scaled for different fields; For example, a quality estimate for an image may be scaled with respect to the average quality in its field, e.g., multiplied with average quality overall divided average quality in the field. Another approach is to combined machine-learning and rule-based quality estimators, wherein the meta-data which is correlated with the accuracy of the image classifier is handled in the rule-based estimator.

In one exemplifying embodiment, of a computer implemented system and/or method for training an image classifier is provided. The image classifier being configured to receive an image as input and to produce a label as output. The method comprising and/or the system being configured for obtaining initial weights for the image classifier, the multiple weights of the image classifier characterizing the image classifier; accessing a training storage comprising multiple training samples, a training sample comprising: a training image, a ground-truth label and meta-data indicating information on the origin of the ground-truth label; and training the image classifier by iteratively; obtaining a training sample from the training storage, applying a quality estimator to the meta-data of the training sample, obtaining a quality estimation of the ground-truth label of the training sample, determining a learning rate from the quality estimation, and applying an iteration of a machine learning algorithm configured with the determined learning-rate to the training-image and ground-truth label, modifying the multiple weights. As pointed out instead of an image other modalities can be used, e.g., technical sensor data. Instead of modulating learning rate, the quality estimate may be used to impact the machine learning in other ways, e.g., by prioritizing or eliminating parts of the training data.

In the various embodiments of system 110, 200, 300, 400 and 500, etc., a communication interface may be included, e.g., selected from various alternatives. For example, the interface may be a network interface to a local or wide area network, e.g., the Internet, a storage interface to an internal or external data storage, a keyboard, an application interface (API), etc. The systems may have a user interface, which may include well-known elements such as one or more buttons, a keyboard, display, touch screen, etc. The user interface may be arranged for accommodating user interaction for starting training of an image classifier, applying an image classifier, training a quality estimator and so on.

Storage, e.g., storage 210 and/or 220, in these systems may be implemented as an electronic memory, say a flash memory, or magnetic memory, say hard disk or the like, or optical memory, e.g., a DVD. Storage may comprise multiple discrete memories together making up the storage. Storage may comprise a temporary memory, say a RAM.

The various systems, e.g., systems 110, 200, 300, 400, 500 may be implemented in a device or in multiple devices. For example, storage and/or processing may be offloaded to cloud services. Typically, these systems comprise one or more microprocessors which executes appropriate software stored at the system; for example, that software may have been downloaded and/or stored in a corresponding memory, e.g., a volatile memory such as RAM or a non-volatile memory such as Flash. Alternatively, the systems may, in whole or in part, be implemented in programmable logic, e.g., as field-programmable gate array (FPGA). The systems may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

In an embodiment, the systems comprise one more circuits configured to implement all or part of an embodiment, e.g., of units described herein. The circuits may be a processor circuit and storage circuit, the processor circuit executing instructions represented electronically in the storage circuits.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

**Figure 6** schematically shows an example of an embodiment of a method for training an image classifier. The image classifier is configured to receive an image as input and to produce a label as output. The image classifier may also produce multiple labels as output. For example, output may be a vector, the components of which indicate the likelihood that a label associated with the component applies to the image. The training may be configured so that the vector sums to 1, though that is not necessary; For example, on label may correspond to 'normal' and other to a specific abnormality.

Method 600 comprises
- obtaining (610) initial weights for the image classifier, the multiple weights of the image classifier characterizing the image classifier,
- accessing (620) a training storage (220) comprising multiple training samples (230), a training sample comprising: a training image (231), a ground-truth label (232) and meta-data (233) indicating information on the origin of the ground-truth label, and
- training (630) the image classifier.
Training the image classifier may comprise iteratively applying:
- obtaining (631) a training sample (230) from the training storage,
- applying (632) a quality estimator to the meta-data of the training sample, obtaining a quality estimation of the ground-truth label of the training sample,
- determining (633) a learning rate from the quality estimation,
- applying (634) an iteration of a machine learning algorithm configured with the determined learning-rate to the training-image and ground-truth label, modifying the multiple weights.

Once the image classifier is sufficiently trained, one may obtain a novel image, say, from an imaging device, and apply the trained image classifier to the novel image. For example, the method may have a training phase for applying learning, e.g., supervised learning, to the image classifier, and a use phase in which the trained image classifier is used to classify novel images. Training and use phases may alternate. For example, the image classifier may be fine-tuned, e.g., receive additional training, on images obtained during the use phase. The fine-tuning may use a similar method as method 600, including obtaining a quality estimation of a label.

Interestingly, having a state-of-the-art image classifier may be used to obtain estimates of the quality of ground-truth labels, which in turn may be used to train a quality estimator. For example, a training method may comprise
- obtaining a first trained image classifier, the first image classifier being configured to receive an image as input and to produce a label as output,
- applying the first trained image classifier to multiple training samples, obtaining a determined label for the multiple training samples,
- comparing the determined label with the ground-truth label to obtain a determined quality estimation,
- training a quality estimator comprising a machine learnable model to predict the determined quality estimation from the corresponding meta-data.
- applying a method to train a second image classifier using the trained training a quality estimator, e.g., method 600.

The first trained image classifier may be comparable to human performance on some types of images, while it may be less good for other types of images. Countermeasures may be taken to avoid that the quality estimator predicts on which images the image classifier do less well, rather than predicting when the domain experts may do less well. For example, the quality estimator may be trained only on meta-data which is correlated to the quality of the domain expert who created the ground truth label, but uncorrelated to the relative quality of the first image classifier for that image; for example, time of day, time of week, years of experience, are suitable inputs, as they do not correlate with the subject matter of the image. For example, the image itself, the domain expert's (sub)specialty (assuming the latter is correlated), are less suited. Other approaches to avoiding correlation with the image classifier may be used. Correlation could be established empirically.

Many different ways of executing the method are possible, as will be apparent to a person skilled in the art. For example, the steps can be performed in the shown order, but the order of the steps may also be varied or some steps may be executed in parallel. Moreover, in between steps other method steps may be inserted. The inserted steps may represent refinements of the method such as described herein, or may be unrelated to the method. For example, step 634 may be executed, at least partially, in parallel for multiple training images. Moreover, a given step may not have finished completely before a next step is started.

Embodiments of the method may be executed using software, which comprises instructions for causing a processor system to perform method 600. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

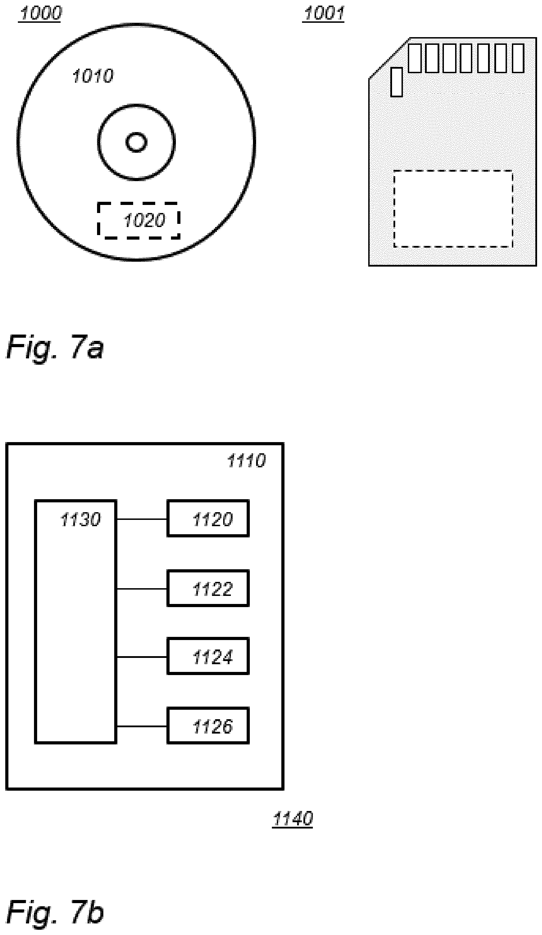

**Figure 7a** shows a computer readable medium 1000 having a writable part 1010, and a computer readable medium 1001 also having a writable part. For example, a computer readable medium may comprise a computer program 1020. Computer readable medium 1000 and 1001 may store data wherein the data indicates one or more of the following:
- instructions, which when executed by a processor system, cause the processor system to perform an embodiment of the training method,
- an image classifier trained according to an embodiment, and/or
- a trained quality estimator trained according to an embodiment.

The computer program 1020 comprises instructions for causing a processor system to perform a training method according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said training method.

**Figure 7b** shows in a schematic representation of a processor system 1140 according to an embodiment of a training system. The processor system comprises one or more integrated circuits 1110. The architecture of the one or more integrated circuits 1110 is schematically shown in Figure 7b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the training system, e.g., device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. Measures recited in mutually different dependent claims can advantageously be combined.

## Claims

1. A computer-implemented training method for a machine-learnable classifier, the classifier being configured to receive sensor data as input and to produce a label as output, the method comprising:
- obtaining (610) initial weights for the classifier, the multiple weights of the classifier characterizing the classifier,
- accessing (620) a training storage (220) comprising multiple training samples (230), a training sample comprising: training sensor data (231), a ground-truth label (232), and meta-data (233) indicating information on the origin of the ground-truth label,
- training (630) the classifier by iteratively,
- obtaining (631) a training sample (230) from the training storage,
- applying (632) a quality estimator to the meta-data of the training sample, obtaining a quality estimation of the ground-truth label of the training sample,
- applying (634) a machine learning algorithm to the training data in dependence on the quality estimation, thus modifying the multiple weights.

2. A computer-implemented training method as in Claim 1, comprising:
- determining (633) a learning rate from the quality estimation,
- applying (634) an iteration of a machine learning algorithm configured with the determined learning-rate to the training sensor data and ground-truth label, modifying the multiple weights.

3. A computer-implemented training method as in any one of the preceding claims, wherein the machine-learnable classifier is an image classifier, the sensor data is an image, training sensor data is a training image, and the machine learning algorithm is applied to the training image.

4. A training method as in Claim 3, wherein the image classifier is a medical image classifier, the ground-truth label indicating a medical abnormality in the image.

5. A training method as in any one of the preceding claims having a training phase configured to train the classifier, and a use phase configured to
- obtaining novel sensor data from a sensor,
- apply the trained classifier to the novel sensor data.

6. A training method as in any one of the preceding claims, comprising:
- obtaining meta-data and a label for novel sensor data,
- obtaining a quality estimation of the label by applying the quality estimator to the meta-data of the training sample, determining a learning rate from the quality estimation, and applying a further iteration of a machine learning algorithm configured with the determined learning-rate to the novel sensor data and corresponding label, modifying the multiple weights.

7. A training method as in any one of the preceding claims, wherein training samples with a high quality estimation are prioritized over training samples with a lower quality estimation.

8. A training method as in any one of the preceding claims, comprising:
- obtaining multiple training samples from the training storage and applying the quality estimator to multiple meta-data of the multiple training samples,
- selecting a batch of training samples from the multiple training samples having a close quality estimate, wherein the machine learning algorithm is applied to the batch of training samples using the same learning rate.

9. A training method as in any one of the preceding claims, wherein the meta-data comprises one or more of:
- information regarding a domain expert who determined the ground-truth label, e.g., specialty, years of experience, user id, user location;
- information indicating the moment in time the ground-truth label was determined, e.g., time of day, day of week, duration of report creation.

10. A training method as in any one of the preceding claims, wherein applying the quality estimator comprises applying a set of rules to the meta-data to compute the quality estimate.

11. A training method as in any one of the preceding claims, wherein a rule in the set of rules is configured to increase or decrease a default quality estimate depending on a favorable or unfavorable element in the meta-data.

12. A training method as in any one of the preceding claims, wherein the quality estimate is determined at least from the time of day the ground-truth label was determined.

13. A training method as in any one of the preceding claims, comprising:
- applying a trained classifier to multiple training samples, obtaining a determined label for the multiple training samples,
- comparing the determined label with the ground-truth label to obtain a determined quality estimation,
- training a quality estimator comprising a machine learnable model to predict the determined quality estimation from the corresponding meta-data.

14. A training method as in claim 13, comprising:
- obtaining a training sample,
- applying the trained quality estimator to the meta-data of the training sample.

15. A system for training a classifier, the classifier being configured to receive sensor data as input and to produce a label as output, the system comprising:
- a communication interface arranged to access a training storage comprising multiple training samples, a training sample comprising: a training sensor data, a ground-truth label and meta-data indicating information on the origin of the ground-truth label,
- a processor circuit configured for
- obtaining initial weights for the classifier, the multiple weights of the classifier characterizing the classifier,
- training the classifier by iteratively,
- obtaining a training sample from the training storage,
- applying a quality estimator to the meta-data of the training sample, obtaining a quality estimation of the ground-truth label of the training sample,
- applying a machine learning algorithm to the training data in dependence on the quality estimation, thus modifying the multiple weights.

16. A system for applying a classifier, the classifier being configured to receive sensor data as input and to produce a label as output, the system comprising:
- a communication interface arranged to obtain novel sensor data from a sensor device, and
- a processor circuit configured to apply a classifier, trained according to any one of claims 1-14 to the novel sensor data.

17. A workstation or imaging apparatus comprising the system of claims 15 and/or 16.

18. A transitory or non-transitory computer readable medium (1000) comprising data (1020), wherein the data indicates one or more of the following:
- instructions, which when executed by a processor system, cause the processor system to perform a method according to any one of claims 1-14,
- a classifier trained according to any one of claims 1-14, and
- a trained quality estimator trained according to claim 13.
